# EUROPEAN PATENT SPECIFICATION

(11) **EP 1 968 926 B1**
(45) Date of publication and mention of the grant of the patent: **02.09.2020**
(21) Application number: 06830049.0
(22) Date of filing: 21.11.2006
(51) Int. Cl.: B01J 2/06, A61K 9/16, A61K 9/20, A61K 9/28, A61K 9/48, A61K 9/50, C07C 29/86

(54) **SPHERICAL MENTHOL PARTICLES**
SPHÄRISCHE MENTHOLPARTIKEL
PARTICULES DE MENTHOL SPHÉRIQUES

(30) Priority: 20.12.2005 US 751629 P
(43) Date of publication of application: 17.09.2008
(73) Proprietor: Symrise AG, 37603 Holzminden (DE)
(72) Inventor: RHEINLÄNDER, Heinz-Dieter, 37639 Lütgenade (DE); NIEKERKEN, Jörg, 37603 Holzminden (DE)
(74) Representative: Fabry, Bernd
(86) International application number: PCT/EP2006/068700
(87) International publication number: WO 2007/071512

(56) References cited:
- WO-A-03/101924
- FR-A1- 2 737 668
- JP-B2- 3 667 791
- US-A1- 2 570 423
- US-A1- 3 123 855
- Unknown: "Untitled", , 1 October 2002 (2002-10-01), XP055553675, Retrieved from the Internet: URL:https://www.google.com/url?sa=t&rct=j& q=&esrc=s&source=web&cd=3&ved=2ahUKEwjFl-X W_angAhUS_qQKHaIgCKQQFjACegQIBBAC&url=http %3A%2F%2Fwww.daega.co.kr%2Fhtml2%2F_admin% 2Fboard_new%2Fkorean%2Fboard_pds_02%2Ffile _download.php%3Fdata%3Dbulk_density_table. pdf&usg=AOvVaw2AtRdvDfuC23iajQf7Dedv [retrieved on 2019-02-07]

## Description

The present invention relates to processes for the preparation of spherical menthol particles.

L-Menthol has a unique refreshing taste, a minty odour and a pronounced cooling effect on the skin and mucosa. It is used, for example, in oral care, in cosmetic and pharmaceutical preparations, in tobacco and in confectionery, as described, for example, in Perfumer&Flavorist, Vol. 13, October-November 1988, p. 37.

L-Menthol is the main constituent of the peppermint oils from Mentha arvensis (content: 70 to 80 %) and Mentha piperita (content: 50 to 60 %). L-Menthol is obtained from the crude peppermint oil by crystallisation. Depending on the crystallisation method and the starting material, the crystals differ in terms of taste and also in terms of the size and shape of the crystals (Perfumer & Flavorist; Vol. 22, November-December 1997, p. 1). A small residual amount of liquid peppermint oil adheres to these menthol crystals obtained from peppermint oils (the content of I-menthol is conventionally not more than 99.2 wt.%) and inhibits caking or clumping of the menthol crystals, but also has a marked influence on the sensory profile.

Many processes for the preparation of synthetic menthol are known. An economic process for the preparation of synthetic I-menthol uses thymol, for example, as starting material. From the eight stereoisomeric menthols formed by hydrogenation, I-menthol is obtained *via* a plurality of process steps in a chemical purity of > 99 % and an enantiomeric purity of > 99 % (e.g. in Bauer, Garbe, Surburg, Common Fragrance and Flavor Materials, 4th Ed., Wiley-VCH, Weinheim 2001, p. 52-55). The I-menthol resulting from this process is distinguished in sensory terms by its purity and intensity.

Menthol, in particular I-menthol, is commercially available in various solid forms; powders, crystals, solidified distillate, flakes and pellets, for example, are conventional.

Synthetic I-menthol (having a melting point of from 42 to 43°C) prepared and crystallised according to the processes of DE-A 2109456, DE-A 2530481 or EP 0 909 205 is commercially available in the form of white crystals or in the form of pellets and also in the form of a solidified distillate (Symrise GmbH & Co. KG, Holzminden).

L-Menthol in flake form is described in US 3,064,311. For its preparation, distilled I-menthol is melted and a thin, molten film layer is applied to a supercooled surface. The solidified I-menthol film is broken up into small parts. The product of this process is a friable I-menthol flake having a thickness of from 0.125 to 1.25 mm and a size of from 3 to 25 mm. The flaked I-menthol so prepared exhibits adhesion and clumping of the flakes after 24 hours, as described in the mentioned US specification.

WO 03/101924 describes menthol pellets having a content of alpha-menthol of greater than or equal to 70 wt.%, which exhibit a comparatively very low tendency to caking or clumping.

FR 2737668 describes the preparation of spherical particles containing lipophilic substances. The particles have a diameter from 0,05 to 5 mm. They are obtained by hot dispersion of a liquid lipophilic phase comprising a lipophilic substance or a mixture of lipophilic substances having a melting point from 25 to 60 °C, optionally a lipophilic active principle and an O/W emulsifier in an aqueous phase comprising water and a hydrophilic polymer in order to obtain a dispersion of globules of lipophilic phase in the aqueous phase, subsequent cooling of this dispersion under agitation to obtain a suspension of the globules, separation of the globules from the suspension and recovery of the globules thus separated. To carry out the hot dispersion step both the lipophilic and the aqueous phase are heated to a temperature at which the lipophilic phase is liquid.

JP 08-020549 relates to I-menthol-containing powder comprising approximately 90 wt.% synthetic I-menthol (particle size from 50 to 200 µm) and approximately 10 wt.% silica gel (particle size less than 10 µm, preferably in the range from 2 to 5 µm). This product (bulk height: 50 cm) did not exhibit caking after one month's storage. For the preparation of the product, a surfactant (e.g. 2 wt.% decaglyceryl monolaurate, based on the total amount of menthol and silica gel) was first introduced into water at a temperature above the melting point of I-menthol (42°C). Silica gel and molten menthol, or menthol on silica gel, were then introduced in succession, at 50°C, into the mixture of water and surfactant. After stirring for a short time, the mixture was cooled with ice-water, washed with water, filtered off and dried.

It is also known that meltable chemical substances that are solid at normal temperature can be granulated by liquefying the substance and introducing the resulting melt into a liquid.

DE 518 090 describes the conversion of salts, in particular fertilisers, into a spherical or sphere-like structure. The process described therein consists substantially in introducing the material to be granulated, in the molten state, dropwise into a cooled, moving liquid in which the material is insoluble. It is necessary for the cooling liquid to be moved tangentially, for example by a blade stirrer, so that a long spiral path (turbulence) forms and a sufficiently long dwell time of the drops of material in the cooling liquid is thereby ensured, so that the spherical or sphere-like structures that subsequently form at the bottom of the cooling vessel are sufficiently solid and do not stick together at the bottom of the cooling vessel.

DE 1 157 202 (corresponding to US 3,123,855) relates to a process for granulating meltable materials, in particular pitch and synthetic resins, in the form of small spherules by allowing a stream of the melt to run into a cooling liquid. This is effected by introducing a stream of the molten material, on which oscillations have been impressed in a manner known *per se,* into a hot cooling liquid with irregular surface tension, preferably water, and guiding the resulting droplets into cooling agent zones having a temperature below the solidification temperature of the material. The stream of molten material preferably runs into the hot cooling liquid beneath the surface thereof. Process variants (which are not preferred) are also described therein, in which the material to be cooled flows from bottom to top and the cooling agent flows downwards in the opposite direction, and also forms of the process in which the material to be cooled and the cooling agent are guided co-currently. In all cases, the temperatures along the stream of material to be cooled, along the path of the granules and along the stream of cooling agent never change suddenly but change constantly according to gradients. In a preferred form, there are added to the cooling agent according to DE 1 157 202 substances that lower the surface/interfacial surface tension of the cooling agent. In the concrete example, pitch (having a softening point of 90°C) heated to 180°C was passed through a nozzle at an operating pressure of 13 atm into a column containing water flowing in counter-current, the water having a temperature of 160°C at the point of introduction of the stream of pitch; at the point of discharge of the resulting pitch spherules at the bottom of the column, which was conical at its bottom end, the water had a temperature of approximately 20°C.

A process for granulating chemical substances, in particular fertilisers, is disclosed in DE 932 246. In that specification, the molten material flowing from a melt container is introduced *via* openings (e.g. perforated bottoms) into a vertically arranged tower-like container which is widened conically in its top portion, the material divided into the form of drops being cooled by an ascending stream of cooling air (in counter-current) to such an extent that the material to be granulated is present at the bottom of the tower-like container in the form of solidified drops.

DE 1 300 514 proposes a process for pelletising sulfur, pitch or meltable plastics, in which the substance in question is introduced dropwise into a cooling-water bath to which there has been added an additive for lowering the adhesiveness of the spherules that form in the cooling-water bath. The additive used therein is a liquid silicone, which is added to the cooling-water bath in an amount of approximately 10 ppm. The addition of the liquid silicones results in substantially quicker solidification of the spherules that are formed (at least at their surface), and as a result the spherules are no longer crushed by other spherules and no longer stick together in larger conglomerates. A particular advantage mentioned in DE 1 300 514 is that the spherules that are formed - under otherwise identical conditions - can be collected and removed from the pelletising vessel at a considerably higher temperature. Furthermore, the granules obtained therein were described as being harder and less friable overall.

According to GB 1,115,071, the material to be granulated, which in that case is specifically a fertiliser, is introduced dropwise, in molten form, into a boiling liquid (cooling liquid), the material to be granulated being substantially insoluble in the boiling liquid. Suitable cooling liquids are in particular organic solvents; water, on the other hand, is not mentioned. It is important that the boiling point of the cooling liquid should be at least 20°C lower than that of the material to be granulated; furthermore, the density of the cooling liquid is preferably lower than that of the molten material to be granulated. According to GB 1,115,071, spherical granules are obtained. In a comparative test (using ammonium nitrate as the material to be granulated), it was further found that when the molten material to be granulated was introduced dropwise into the same cooling liquid that was not boiling (at a temperature of 30°C), with simultaneous stirring of the cooling liquid, irregularly shaped, that is to say non-spherical, granules were obtained.

In the so-called wet prilling process, a (sulfur) melt is allowed to flow or drip through one or more perforated plates (nozzle floors) into a prilling container or prilling tower filled with water, or is atomised through a nozzle lance (US 3,637,351).

EP 0 064 311 relates to a wet prilling process for low-melting substances, for example substances that are insoluble in water, in particular for sulfur, by allowing a melt of the substance to be prilled to pass through perforated plates first into a gaseous environment, with the formation of individual drops, and then into a cooling liquid (e.g. water), in which the prills cool and solidify. Removal of the prills is carried out in EP 0 064 311 by means of a propulsion jet pump located at the bottom end of the prilling tower, which pump is intended to permit very gentle conveying of the prills. According to EP 0 064 311, the distance between the surface of the cooling liquid and the melt feed device in the prilling tower is optimally adjusted when drop formation and drop removal take place within the melt streams and the prill particles are neither comminuted nor greatly deformed when they come into contact with the virtually still surface of the cooling liquid. In wet prilling processes, spherical granules are conventionally obtained, the surface properties of which are influenced *inter alia* by the additives (e.g. surfactants) present in the cooling medium.

IN 157 628 describes the preparation of menthol granules in water. In that process, water and solid menthol are first introduced into a stirred vessel in a weight ratio in the range of from 2:1 to 25:1, and then the contents of the vessel are heated (with stirring) to a temperature in the range of from 40 to 60°C so that a dispersion of molten menthol in water is present (melting point of I-menthol: 42-43°C, dl-menthol: 38°C). The menthol/water dispersion is cooled to a temperature in the range of from 10 to 25°C, with stirring at stirrer speeds in the range of from 50 to 500 rpm, so that granules having a mean diameter in the range of from 1 to 10 mm form from the menthol droplets by solidification (the cooling water used in the cooling-water circuit has a temperature in the range of from 2 to 10°C). The contents of the vessel are then maintained at that temperature for a further 30 to 60 minutes, before the water is separated off and the granules are dried. A disadvantage of the process according to IN 157 628 is that large amounts of energy are required to heat and then cool the water that is present, and a long dwell time of the menthol, or of the granules prepared therefrom, is necessary as a result of the long cooling phase. Because of the mechanical stress and turbulence caused to the menthol droplets by the stirrer mechanism, very irregular granules of non-uniform shape are obtained. Furthermore, our own tests have shown that the granules prepared according to IN 157 628 have a water content of up to 4 wt.% after a drying time of 24 hours, the particle size distribution is very broad, and an undesirable fines content is obtained (with regard to further differences, reference is made at this point to the comments made hereinbelow; a direct comparison of the process according to the invention, or of the menthol granules according to the invention, and the process or granules according to IN 157 628 is to be found hereinbelow in Example 5).

The object of the present invention was to provide menthol particles that are as spherical and as uniform as possible, as well as processes and devices for the preparation thereof. The menthol particles (menthol granules) are to possess improved, high storage stability, are to be simple to prepare and easy to handle, and are to have a low tendency to caking.

According to the present invention, the stated object is achieved by a process according to claim 1.

The term "menthol drops" refers to molten menthol in drop form.

The term "spherical menthol particles" refers to menthol particles having a sphericity (ratio of the surface area of a sphere of equal volume to the actual surface area; sphericity according to Wadell) of at least 0.8.

The temperature of the menthol melt during the metering is in the range of from 42 to 60°C, more preferably in the range of from 45 to 55°C. After metering into the water, which has a markedly lower temperature, the menthol melt, which according to the invention assumes the form of menthol drops in the water, cools until the menthol drops finally solidify in the water to give the spherical menthol particles.

The water used can be distilled or demineralised water, but the use of drinking water is preferred; the water can also contain small amounts of dissolved substances.

According to the invention, the water into which the menthol melt is metered has a temperature in the range of from 0 to 12°C. The water temperature is preferably greater than 0°C, the temperature range from 2 to 10°C being preferred and the temperature range from 4 to 8°C being particularly preferred. The maintenance of the temperature range according to the invention is critical for the success of the process according to the invention. With an otherwise identical test arrangement, comparative tests using water at a temperature in the range of from 15 to 17°C yielded granules that exhibited a sponge-like, highly branched structure and did not possess spherical geometry.

When water having a temperature in the range of from 2 to 10°C is used, spherical menthol particles are obtained which, compared with menthol particles obtained at a water temperature of from 10 to 12°C, possessed even greater sphericity.

The manner in which the menthol melt is introduced into the water can be varied; according to the invention, however, menthol drops should temporarily be present in the water after the metering, which menthol drops then solidify in the water to give the spherical menthol particles. In particular, the menthol melt (a) can be metered into the water in the form of drops or (b) can be metered into the water in the form of a stream, in such a manner that the stream breaks up in the water to form the drops. Alternative (b) is preferred.

Very particular preference is given to an embodiment in which the menthol melt is introduced directly (that is to say without passing through a gaseous medium for drop formation, see EP 0 064 311) through one or more openings into the (cooling) water, which is preferably at a specific temperature. The menthol melt is preferably metered into the water in the form of a stream, and oscillations, for example in the range of from 2 to 120 hertz, preferably in the range of from 10 to 60 hertz, are preferably imposed/impressed on the molten menthol stream (e.g. by pressure fluctuations). Alternatively, a stream of molten menthol is preferably metered into the cooling water through openings (for example a perforated plate having one or more bores), the stream being introduced under a pressure that is slightly higher than the pressure caused by the column of water located above. In each of the preferred alternative cases, drops of molten (liquid) menthol form in the water, which drops then solidify to give the spherical menthol particles.

The spherical form of the menthol particles is given by the drop form, present according to the invention, of the menthol in the water; in the preferred embodiment of the process according to the invention, therefore, it is given by the decomposition of the stream of molten menthol into drops. The drops typically ascend upwards during cooling and solidification (hardening) without the influence of shear forces (e.g. a stirrer mechanism). The spherical form that forms in the water is retained until solidification to the spherical menthol particles. During solidification, the menthol particles do not rub against one another to an appreciable degree; occasional contact is not critical for the result of the process.

In the process according to the invention, only the heat that is released during cooling and solidification of the menthol is removed from the water. The menthol droplets that form at the nozzle are able to form unhindered, so that the inclusion of water is avoided to the greatest possible extent.

It is possible for the process according to the invention to be carried out without movement of the (cooling) water, in which case cooling of the water is preferably achieved by means of jacket cooling; alternatively, the (cooling) water can be moved, preferably in such a manner that a flow (in particular an upward flow) is achieved, with which the menthol drops (or the menthol particles obtained therefrom by solidification) are transported co-currently.

Accordingly, preference is given according to the invention to a process in which
(a) the menthol melt is metered into the water in such a manner that the menthol drops ascend upwards in the water (e.g. owing to their low density as compared with water)
   and/or
(b) the water is made to flow and the menthol melt is metered into the flowing water in such a manner that the menthol drops are transported with the flow of water, preferably transported upwards, more preferably upwards in vortex or spiral form.

When in a process according to the invention the menthol drops ascend upwards in the water (in still or moving water), the mean rate of ascent of the menthol drops (menthol particles) within the ascent path is preferably in the range of from 3 to 20 cm/s, more preferably from 5 to 15 cm/s. At the same time or alternatively, it is preferred to establish a mean dwell time for the menthol drops or particles in the water in the range of from 2 to 120 seconds, preferably from 5 to 60 seconds, particularly preferably from 5 to 30 seconds.

The solidified spherical menthol particles are preferably left in the water at least until their surface temperature is not more than 20°C. Depending on the size of the menthol particles, which in turn is dependent on the size of the menthol drops (before solidification) and accordingly optionally also on the width of the stream of molten menthol, dwell times that are within the above-indicated range of preferred mean dwell times are generally sufficient.

The "diameter" of a menthol particle (according to the invention) refers hereinbelow to the arithmetic mean of the largest and smallest diameter of the particle. Depending on the manner in which the menthol melt is metered into the (cooling) water, that is to say, for example, depending on the diameter of the openings of, for example, a perforated plate, a nozzle (plate) or a feed nozzle, the diameter of the menthol particles that can be prepared by the process according to the invention is preferably in the range of from 0.3 mm to 10 mm, more preferably in the range of from 0.5 to 8 mm, particularly preferably in the range of from 1 to 5 mm.

The diameter of the menthol particles can additionally also be varied by the speed of entry into the cooling liquid (metering speed), which is given by the admission pressure at the metering nozzle (or the menthol throughput) and the chosen nozzle cross-section.

For apparatus-related reasons, it is preferred to place the water in a vessel that has a base and then to meter the menthol melt into the water from the base. In particular the process steps described above, in which the molten menthol is introduced into the (cooling) water through openings, can then be converted particularly simply in terms of apparatus.

The spherical menthol particles prepared by the process according to the invention are remarkably storage-stable and, even after a prolonged storage period, are still capable of flowing, can be handled comfortably and safely, and are free-flowing, pourable and meterable. Owing to the uniform spherical geometry and size distribution, in particular compared with products according to IN 157 628, the spherical menthol particles prepared by the process according to the invention have a lower tendency to caking, and any caked spherical menthol particles come apart more easily.

In a process according to the invention, in particular according to a preferred embodiment as described above, the spherical menthol particles are preferably separated from the (cooling) water (see in this connection also the comments made hereinbelow in relation to preferred embodiments of devices according to the invention) and then dried to a moisture content of less than 1 wt.%, preferably less than 0.5 wt.%, based on the total weight of the menthol particles.

In a process according to the invention, the water is preferably placed in a vessel having an overflow, and the spherical menthol particles are discharged from the vessel through the overflow together with water. Such a form of the process is particularly simple (see also the comments made hereinbelow in relation to preferred devices according to the invention).

The addition of an agent that lowers the surface/interfacial surface tension, such as, for example, a surfactant, and/or of liquid silicones to the (cooling) water is not necessary and also not advantageous in a process according to the invention. The (cooling) water used in a process according to the invention is therefore preferably free of agents that lower the surface/interfacial surface tension, in particular free of surfactants, and/or free of liquid silicones.

In a process according to the invention, fresh water having a temperature in the range of from 0 to 12°C is preferably introduced continuously into the region in which the menthol melt is to be metered in. At the same time, water is preferably removed from the system, likewise continuously, which water has been heated owing to the loss of heat from the menthol melt (menthol drops, menthol particles). The (cooling) water is preferably guided in a circuit, advantageously taking heat from the menthol melt, in a vessel in which the menthol melt is metered into the water, and being cooled outside the vessel.

The separation of (cooling) water, which is preferably guided in a circuit, and menthol particles can be carried out by separating techniques known *per se,* for example by sieving, filtration (continuous or discontinuous), centrifugation or the like.

Further preferred embodiments of the process according to the invention will become apparent from the following description, the Examples and the accompanying patent claims.

The present invention relates also to a device for carrying out the process according to the invention; such a device comprises;
- means for melting menthol and for heating the molten menthol to a temperature in the range of from 42 to 60°C (melting and heating means),
- a vessel for receiving water, having a base, and
- means, associated with the vessel (and preferably also connected to the melting and heating means), for metering the molten menthol having a temperature in the range of from 42 to 60°C (a) in the form of drops or (b) In the form of a stream into the water from the base.

It will be understood that the dimensions (length or height) of the (cooling) vessel provided to receive water are given substantially by the requirements in respect of the necessary dwell time for the menthol drops/menthol particles. The dwell time is in practice so chosen that adequate crystallisation of the menthol granules is ensured, which in turn is generally the case when the surface temperature of the spherical menthol particles is not more than 20°C (see above). The person skilled in the art will take this requirement into consideration when choosing a suitable vessel, as well as the fact that the means for metering the molten menthol into the water from the base affect the size of the menthol drops present in the water, which in turn has an effect on the required dimensions of the vessel (because the rate of ascent of the menthol drops and their solidification behaviour are dependent on the drop size).

A device according to the invention for carrying out the process according to the invention preferably comprises means for monitoring the water temperature in the vessel. The device further comprises means for setting a water temperature in the range of from 0 to 12°C in the vessel at least at the site of metering.

It is also advantageous for the vessel of such a device to have an overflow. See in this connection the comments made in relation to the corresponding process according to the invention.

Such a device additionally advantageously comprises means for separating spherical menthol particles that have formed from the (cooling) water. Such a separating device can be, for example, a sieve or filter, see in this connection the Examples hereinbelow.

The vessel for receiving water is advantageously an upwardly extending pipe having a base. During operation, the pipe is filled with (cooling) water. The means for metering molten menthol into the water are located in or at the base of the pipe. Menthol melt that has been metered in forms menthol drops, which ascend inside the vessel and thereby solidify.

The device preferably comprises means for making the water flow, preferably upwards in vortex or spiral form. In that case, the menthol drops in the device are transported with the flow of the water, preferably upwards, preferably in vortex or spiral form. For example, the means for making the water flow can comprise a water inlet with which water can be introduced into the vessel, for example, tangentially with respect to the vessel wall, so that, for example, ascending turbulence of the column of water present in the vessel is formed.

Further preferred embodiments of devices follow from the above explanations relating to the process according to the invention. For carrying out preferred process steps, corresponding means are, of course, present in a corresponding device.

The present description relates also to a spherical menthol particle and to an amount (number) of spherical menthol particles obtainable by a process according to the invention. For the term "spherical menthol particles", see the definition given hereinbefore.

The quotient d/D of the smallest and largest diameter in at least 80 % of the menthol particles of the amount, preferably in at least 90 % of the menthol particles of the amount, is in the range 1.00 ≥ d/D ≥ 0.80.

It is particularly preferred if the quotient d/D of the smallest and largest diameter in at least 80 % of the menthol particles of the amount, preferably in at least 90 % of the menthol particles of the amount, is in the range 1.00 ≥ d/D ≥ 0.90, more preferably in the range 1.00 ≥ d/D ≥ 0.95.

The process according to the invention permits the preparation of such preferred amounts of spherical particles, that is to say the preparation of very uniformly shaped spherical menthol particles (menthol granules) which, unlike menthol pellets, do not have a moulding seam.

The particle size distribution is conventionally very narrow and the fines content, where present, is very low. See in this connection also the Examples hereinbelow. The bulk density of an amount of spherical menthol particles preferably has a value greater than 500 g/litre. When the process according to the invention is carried out there is conventionally obtained an amount according to the invention of spherical menthol particles in which the Mohs hardness of the individual spherical menthol particles is at least 2. Such spherical menthol particles are harder than menthol particles (menthol granules) as are obtainable according to IN 157 628; see in this connection also the Examples hereinbelow.

The residual moisture of an amount of spherical menthol particles is preferably less than 1 wt.%, more preferably less than 0.5 wt.%, based on the total weight of the amount of spherical particles including residual moisture. See in this connection the comments made in relation to the preferred embodiment of a process according to the invention hereinbefore and the Examples hereinbelow.

Particular preference is given to an amount of spherical menthol particles, obtainable by the process according to the invention, in which
- the bulk density thereof is greater than 500 g/litre,
- the quotient d/D of the smallest and largest diameter in at least 80 % of the menthol particles of the amount is in the range 1.00 ≥ d/D ≥ 0.8, preferably in at least 90 % of the menthol particles of the amount,
- the Mohs hardness of the spherical menthol particles is at least 2, and
- the residual moisture of the spherical menthol particles is less than 1 wt.%, preferably less than 0.5 wt.%, based on the total weight of the amount of spherical particles including residual moisture.

Spherical menthol particles described herein exhibit a significantly smoother surface as compared with the menthol particles such as are obtainable according to IN 157 628, as a result of which the strongly pronounced sublimation properties of menthol are significantly reduced. A further result thereof is that the spherical menthol particles described herein possess a markedly reduced tendency to caking as compared with the menthol particles such as are obtainable according to IN 157 628.

The spherical menthol particles described herein having the same size and under the same dissolution conditions, dissolve markedly more quickly than menthol pellets (that is to say menthol granules compressed under pressure).

The spherical menthol particles described herein which are obtainable by a process according to the invention, can be prepared from d-menthol, I-menthol and any desired mixtures of d-menthol and I-menthol, with I-menthol, d-menthol and racemic menthol being preferred and I-menthol being particularly preferred.

The menthol used in the process according to the invention can be of synthetic or natural origin, but the use of synthetic menthol is preferred. In particular when using menthol of natural origin, it is difficult to ensure the high purity of the menthol that is often desired.

It is also possible to use any desired mixtures of synthetic and natural menthol and of racemic and enantiomerically pure menthol.

In comparison with the menthol products obtained according to the processes disclosed in the documents cited above, the menthol particles described herein are particularly advantageous in sensory terms. In particular, the menthol particles described herein appear to be fresher, more intense and clearer in terms of odour and, in particular, taste.

Accordingly, the present description relates also to articles comprising a menthol particle as described herein or an amount of spherical menthol particles. The articles are preferably selected from the group consisting of: products suitable for consumption, smokers' products, tobacco products, perfumes (fragrance mixtures), flavouring mixtures, oral hygiene products, cosmetic, pharmaceutical and dermatological products, encapsulated or coated menthol particles according to the invention.

A related aspect of the present description relates to the use of a menthol particle as described herein or of an amount of spherical menthol particles in the preparation of a menthol-containing article, in particular of an article selected from the group consisting of: products suitable for consumption, smokers' products, tobacco products, perfumes (fragrance mixtures), flavouring mixtures, oral hygiene products, cosmetic, pharmaceutical and dermatological products, encapsulated or coated menthol particles.

The spherical menthol particles described herein are used in particular for flavouring or fragrancing the mentioned articles.

The amount of menthol particles described herein in a product varies considerably depending on the type of product:
In ready-for-use products, such as, for example, oral hygiene products, products suitable for consumption (e.g. foodstuffs) or cosmetic products, the amount of menthol particles described herein is preferably in the range of from 0.01 to 10 wt.%, particularly preferably in the range of from 0.1 to 5 wt.%, based on the total weight of the ready-for-use product.

In flavourings or fragrance mixtures, on the other hand, the amount of menthol particles described herein can be very much higher and is generally in the range of from 0.01 to 70 wt.%, preferably in the range of from 1 to 50 wt.%, based on the total weight of the flavouring or fragrance mixture.

A product suitable for consumption is a product that is intended to be introduced into the human oral cavity, to remain there for a particular time and then to be either swallowed, that is to say consumed (e.g. foodstuffs), or removed from the oral cavity again (e.g. chewing gums). Also included are all substances or products that are intended to be ingested by humans in the processed, partially processed or unprocessed state. Further included are all substances that are added to the product suitable for consumption during its preparation, processing or treatment.

Preferred products suitable for consumption are, for example, baked goods (biscuits, cakes, muffins, waffles, baking mixtures), sugar products (hard caramels, soft caramels, chewy sweets, compressed products, dragees, sugar pearls, sugar fillings), milk products (yoghurts, puddings, ice cream), chocolate products (white, milk or dark chocolate, chocolate bars), fatty substances (fillings for baked goods, such as, for example, fillings for biscuits, fatty fillings for chocolate, fatty fillings for bars), chewing gums (sugar-free, sugar-containing, strips, compressed products, dragees), snacks and snack mixtures, water-soluble powdered products, toppings.

An oral hygiene product (also referred to herein below as an oral care product or oral hygiene preparation) is understood as being one of the formulations known to the person skilled in the art for cleaning and caring for the oral cavity and the pharynx and also for freshening the breath. Care of the teeth and gums is also expressly included herein. Forms of administration of conventional oral hygiene formulations are creams, gels, pastes, foams, emulsions, suspensions, aerosols, sprays, as well as capsules, granules, pastilles, tablets, sweets or chewing gums.

Preferred oral hygiene products are in particular tooth care agents, such as toothpastes, tooth creams, tooth gels, tooth powders, mouthwashes, dental floss, seamless capsules, sweets for sucking, and sugar-free chewing gums.

A menthol particle as described herein can be processed further in particular by encapsulation. Preferably, the menthol particle described herein and/or a liquid or solid preparation containing it is encapsulated with a solid encapsulating material which is preferably selected from starches, degraded or chemically or physically modified starches (in particular dextrins and maltodextrins), gelatines, gum arabic, agar-agar, ghatti gum, gellan gum, modified and unmodified celluloses, pullulan, curdlan, carrageenans, alginic acid, alginates, pectin, inulin, xanthan gum and mixtures of two or more of the mentioned substances.

Cosmetic and/or dermatological products comprising one or more spherical menthol particles as described herein can otherwise be composed in the conventional manner and be used for cosmetic and/or dermatological sun protection, also for the treatment, care and cleansing of the skin and/or hair and as a make-up product in decorative cosmetics. Accordingly, the products, depending on their composition, can be used, for example, as a skin protection cream, cleansing milk, sun protection lotion, nutrient cream, day or night cream, etc. It is optionally possible and advantageous to use the products as a base for pharmaceutical products. Preference is given in particular to those cosmetic and dermatological products that are in the form of a skin care or make-up product. Typical forms are creams, gels, lotions, alcoholic and aqueous/alcoholic solutions, emulsions, or preparations in stick form. Such agents can also comprise as further auxiliary substances and additives mild surfactants, co-emulsifiers, superfatting agents, pearlescent waxes, consistency-imparting agents, thickeners, polymers, silicone compounds, fats, waxes, stabilisers, biogenic active ingredients, deodorant active ingredients, anti-dandruff agents, film-forming agents, swelling agents, hydrotopic agents, preservatives, insect repellents, tanning agents, artificial self-tanning agents (e.g. dihydroxyacetone), solubilisers, perfume oils, colourings, germ-inhibiting agents and the like.

For use, the cosmetic and dermatological products are applied in a sufficient amount to the skin and/or hair in the manner conventional for cosmetics. Particular preference is given to those cosmetic and/or dermatological products that are in the form of a cosmetic agent for protecting the skin and hair. In addition to UV-A, UV-B and/or broad-band filters, such agents can advantageously contain at least one inorganic pigment, preferably an inorganic micropigment.

The cosmetic and/or dermatological products can contain cosmetic auxiliary substances such as are conventionally used in such products, for example preservatives, bactericides, perfumes, substances for preventing foaming, colourings, pigments that have a colouring action, thickeners, moisturising and/or humectant substances, fats, oils, waxes or other conventional constituents of a cosmetic or dermatological product, such as alcohols, polyols, polymers, foam stabilisers, electrolytes, organic solvents or silicone derivatives.

The invention will now be explained in greater detail first with reference to the accompanying figure, in which:

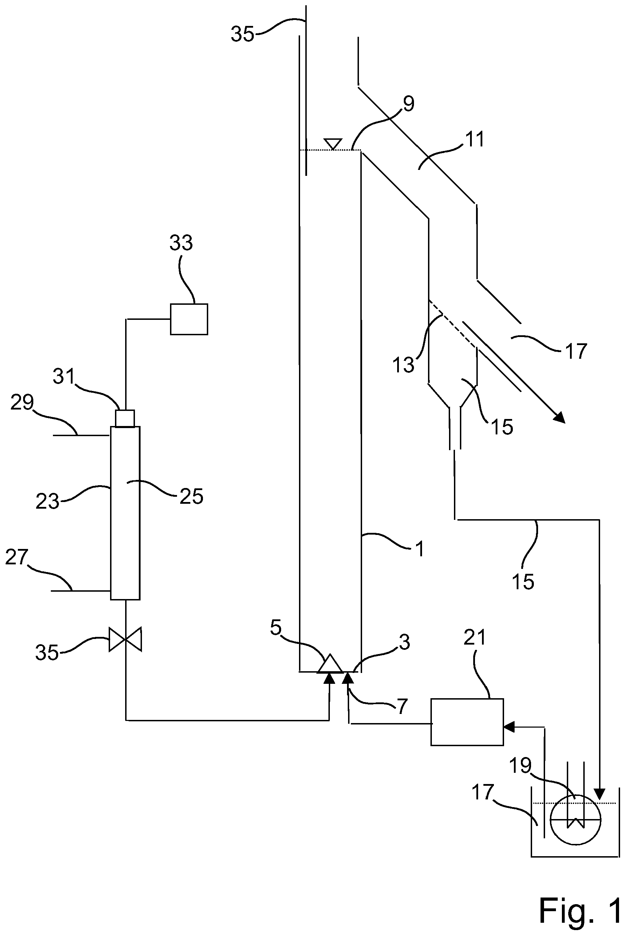

- Figure 1: is a diagrammatic representation of a device for carrying out the process according to the invention.

The device comprises a tubular vessel 1 for receiving water, having a base 3. Associated with the base 3 is a metering nozzle 5, which serves to meter in molten menthol from the base. Also associated with the base 3 of the vessel 1 is a water inlet 7, through which water having a temperature in the range of from 0 to 12°C can be introduced into the tubular vessel.

The vessel 1 further comprises an overflow 9, with which there is associated an outlet 11 which leads to a separating device 13, which is a water filter. In the separating device 13, the outlet 11 forks into a water return 15 and a product discharge 17. The water return 15 leads to a water container 17 equipped with a cooling device, in particular a heat exchanger 19. From the water container 17, water can be passed by means of a water pump 21 into the vessel 1 through the inlet 7 in the base 3 of the vessel 1.

Finally, a thermometer 35 is also associated with the vessel 1, which thermometer 35 is arranged for measuring the water temperature just below the overflow 9.

The device comprises a melting pipe 23 which is heated *via* a water-heating jacket 25 (having a hot water inlet 27 and a hot water outlet 29). The melting pipe 23 is filled with menthol *via* a product inlet 31. Associated with the melting pipe 23 are a pressure transducer 33 and a metering valve 35.

For the preparation of menthol particles according to the invention, the vessel 1 is filled with water so that a column of water extending between the base 3 and the overflow 9 is formed. The water to be introduced into the vessel 1 is taken from the water container 17, which is not shown to scale in Figure 1, in which it is adjusted by means of the heat exchanger 19 to a temperature in the range of from 0 to 12°C. From the water container 17, the water is transported by means of the water pump 21 through the inlet 7 into the vessel 1. The temperature of the water in the vessel 1 is monitored by means of the thermometer 35. As soon as the water level reaches the height of the overflow 9, water flows out of the vessel 1 through the overflow 9 into the outlet 11. From there, it passes through the separating device (water filter) 13 into the water return 15 and from there back into the water container 17, where it is cooled; during operation, the water is guided in a circuit.

During operation of the device, menthol is introduced into the melting pipe 23 *via* the product inlet 31. In the melting pipe 23, the menthol is melted by the heating jacket 25, which is filled with hot water which flows into the heating jacket 25 through the hot water inlet 27 and out of it again through the hot water outlet 29. From the melting pipe 23, molten menthol, in an amount dependent on the setting of the transducer 33, the metering valve 35 and the chosen metering nozzle 5, passes into the tubular vessel 1 from the base. When the molten menthol, for example in the form of a stream, has entered the column of water already present in the vessel 1, the stream of menthol (line of menthol) breaks up into individual (liquid) drops of menthol. Because of their low density and because of the flow resulting from the continuous supply of water from the water container 17, the drops ascend upwards, whereby they solidify and solid spherical menthol particles form. Finally, these particles reach the overflow 9 and are transferred with water to the outlet 11 of the device. While the water - as stated above - passes into the water return 15 when it comes into contact with the water filter (as an example of a separating device), the spherical menthol particles are prevented from doing so and are thus separated from the water. The spherical menthol particles pass into the product discharge 17 and thereby follow the direction indicated by the arrow shown in Figure 1. The water that has passed into the water return 15, which is passed into the water container 17, is cooled again therein and is again introduced into the vessel 1 by means of the water pump 21. The (cooling) water is accordingly guided in a circuit.

The Examples which follow explain the invention. Unless indicated otherwise, all amounts are by weight.

### Example 1

Water is placed in a vertically arranged tubular container having a capacity of 5 litres and is adjusted to a temperature of 5°C by circulation by pumping. 700 g of menthol are melted and heated to 50°C. Through an opening (perforated plate) having a diameter of 0.8 mm in the base of the container, the menthol is metered in the form of a stream, with a low admission pressure of 1140 mbar, into the water. The liquid menthol stream breaks up into drops, which slowly ascend upwards in the column of water and thereby solidify. The dwell time of the ascending drops is 13 seconds, determined by the height of the container of 1.2 m. The drops, which have solidified to granules, are removed *via* an overflow and then dried in the air (20°C, with a humidity of 50 %) for 24 or 48 hours. The resulting amount of spherical menthol particles has a water content of less than 1 % and possesses the following particle size distribution:

### Particle size distribution:

> 2.5 mm = 71 %
> 1.25 mm = 27 %
> 1 mm = 1.5 %
> 0.8 mm = 0.5 %
> 0.5 mm = 0 %
> 0.2 mm = 0 %
< 0.2 mm = 0 %

### Example 2

Water is placed in a vertically arranged tubular container having a capacity of 5 litres and is adjusted to a temperature of 5°C by circulation by pumping. In addition, water at a specific temperature is fed to the container *via* an opening that runs in tangentially with respect to the wall of the container, resulting in ascending turbulence of the column of water. 700 g of menthol are melted and heated to 50°C. Through an opening having a diameter of 0.8 mm in the base of the container, the menthol is metered in the form of a stream, with an admission pressure of 1215 mbar, into the water. The liquid menthol stream breaks up into fine drops, which are carried along in the water vortex, ascend upwards and thereby solidify. The dwell time of the ascending menthol particles is 24 seconds, determined by the height of the container of 1.2 m and the strength of the turbulence. The drops, which have solidified to granules, are removed *via* an overflow and then dried in the air (20°C, with a humidity of 50 %) for 24 hours. The resulting amount of spherical menthol particles has a water content of less than 1 % and possesses a particle size distribution comparable to that of Example 1.

### Example 3

Water is placed in a vertically arranged tubular container having a capacity of 7 litres and is adjusted to a temperature of 13°C by circulation by pumping. In addition, water at a specific temperature is supplied to the container *via* four openings in the base each having a diameter of 10 mm, as a result of which a uniformly ascending flow is formed in the column of water. 700 g of menthol are melted and heated to 50°C. Through three openings (distance between the openings: 2 cm) each having a diameter of 0.8 mm in the base of the container, the menthol is metered in the form of a stream, with a low admission pressure of 1140 mbar, into the water. The liquid menthol streams break up into drops, which slowly ascend upwards in the column of water and thereby solidify. The dwell time of the ascending drops is 16 seconds, determined by the height of the container of 1.6 m. The drops, which have solidified to particles, are removed *via* an overflow and then dried in the air (20°C, with a humidity of 50 %) for 24 hours. The resulting amount of spherical menthol particles has a water content of less than 1 % and possesses a particle size distribution comparable to that of Example 1.

### Example 4: Comparative Example (based on Example 1 of IN 157 628)

940 g of water and 188 g of I-menthol were placed together in a double-walled reactor and heated to 49-50°C with stirring (propeller stirrer, 580 rpm). The mixture was maintained at 49-50°C for 15 minutes, with stirring, and then cooled to 12°C by means of cooling water at 5°C in the jacket cooling system, with stirring, and maintained at that temperature for 40 minutes. The resulting menthol granules (particles) were then separated from the water. For more rapid drying, the granules can be spun-dry at 3000 rpm for 5 minutes, the water content of the granules after the spin-drying operation was 8.9 %.

Note: Unlike in Example 1 of IN 157 628, it was necessary to set a stirrer speed of at least 550 rpm because, below that speed, a large amount of menthol was deposited on the stirrer apparatus on drying, which led to such a great disequilibrium that the tests had to be discontinued. Furthermore, it was found to be necessary, in order to achieve more uniform granule formation in respect of size distribution and geometry, to inoculate finely crystalline menthol (crystal sizes in the range from 0.5 to 1 mm) during cooling. Without inoculation, on the other hand, even coarser, even more unevenly shaped granules were obtained.

Water contents after drying at 20°C, without air circulation, at a humidity of 50 %:

| | |
|---|---|
| after 24 hours | 4.0 % |
| after 48 hours | 2.15 % |

Particle size distribution of the dried granules after 48 hours
> 2.5 mm = 90 %
> 1.25 mm = 6.1 %
> 1 mm = 1.3 %
> 0.8 mm = 0.9 %
> 0.5 mm = 1.0 %
> 0.2 mm = 0.3 %
< 0.2 mm = 0.4 %

### Example 5: Comparison of properties

Particles obtained according to the invention in comparison with granules according to IN 157 628

| **Property** | **Example 1 according to the invention, see above** | **(Comparative) Example 4, see above,** |
|---|---|---|
| | | **analogous to IN 157 628** |
| Bulk density | 540 g/litre | 432 g/litre |
| Roundness | Substantially spherical | Irregular granules |
| | Quotient of smallest and largest diameter approx.: 0.99 > d/D > 0.8 | Quotient of smallest and largest diameter approx.: 0.90 > d/D > 0.5 |
| | Interpretation: | Interpretation: |
| | The shape is given by the breaking up of the stream of liquid, which enters the water directly, into drops. The drops are kept in suspension in the water and ascend upwards during hardening without the influence of shear forces. The round drop form is retained. The particles do not rub against one another during hardening. | The shape is given by the breaking up of the menthol phase as a result of the mechanical shear forces produced by the stirring device. Drops are therefore without shape, and collisions of individual drops lead to non-round granules. Breaking up occurs in the post-cooling phase as a result of collisions of the particles with one another and with the stirring device. |
| Surface roughness | Smooth, not very amorphous | Rough, highly amorphous |
| | on prolonged storage, sight blooming at the surface is possible as a result of sublimation | on prolonged storage, pronounced blooming at the surface is possible as a result of sublimation |
| Particle size distribution (mm) | > 2.5 = 71 % | > 2.5 = 90 % |
| | > 1.25 = 27 % | > 1.25 = 6.1 % |
| | > 1 = 1.5 % | > 1 = 1.3 % |
| | > 0.8 = 0.5 % | > 0.8 = 0.9 % |
| | > 0.5 = 0 % | > 0.5 = 1.0 % |
| | > 0.2 = 0 % | > 0.2 = 0.3 % |
| | < 0.2 = 0 % | < 0.2 = 0.4 % |
| | | Large granules with fines content |
| Hardness (according to Mohs) | 2 = can be scratched with the fingernail can scarcely be rubbed between the fingers | 1 = can be scratched with the fingernail can easily be rubbed between the fingers |
| Sensitivity to rubbing | Low, owing to higher hardness and spherical, smooth surface form | High, owing to lower hardness and rougher, more uneven surface |
| Freedom from dust | Markedly lower dust content, small pearls may be possible, depending on the nozzle stream, as a result of so-called side stream, no dust formation on water crystallisation | Marked dust content as a result of mechanical friction during processing (see above) |
| Clumping behaviour | Clumps readily with time owing to sublimation and adhesion at the contact surface between the pearls, but readily separated because the contact area is very small on account of the spherical shape. More advantageous surface area/volume ratio with the same particle size. | Clumps considerably with time owing to sublimation and adhesion at the contact surface between the granule particles, clumped products difficult to separate because the contact area is larger owing to the irregular shape. |
| | | Less advantageous surface area/volume ratio with the same particle size. |
| Residual moisture after 24 hours' drying time | 0.4 wt.% | approx. 4 wt.% |
| Residual moisture after 48 hours' drying time | 0.2 wt.% | approx. 2 wt.% |

### Formulation examples:

### F1. Tooth cream against plaque

| | I (%) | II (%) | III (%) |
|---|---|---|---|
| Na carboxymethylcellulose | 1.00 | 1.00 | 1.00 |
| Glycerol | 12.50 | 12.50 | 12.50 |
| Sorbitol, 70 % in water | 29.00 | 29.00 | 29.00 |
| Na saccharinate | 0.20 | 0.20 | 0.20 |
| Na fluoride | 0.22 | 0.22 | 0.22 |
| Azacycloheptane 2,2-diphosphonic acid, disodium salt | 1.00 | 1.00 | 1.00 |
| Bromochlorophene | 0.10 | 0.10 | 0.10 |
| Spearmint flavour | 1.00 | 0.60 | 0.20 |
| Menthol particles from Example 1 | 0.125 | 0.56 | 1.00 |
| Abrasive silica | 15.00 | 15.00 | 15.00 |
| Thickening silica | 5.00 | 5.00 | 5.00 |
| Sodium dodecylsulfate (SDS) | 1.50 | 1.50 | 1.50 |
| Dist. water | ad 100.00 | ad 100.00 | ad 100.00 |

### F2. Tooth cream for the care of sensitive teeth

| | I (%) | II (%) | III (%) |
|---|---|---|---|
| Na carboxymethylcellulose | 0.70 | 0.70 | 0.70 |
| Xanthan gum | 0.50 | 0.50 | 0.50 |
| Glycerol | 15.00 | 15.00 | 15.00 |
| Sorbitol, 70 % in water | 12.00 | 12.00 | 12.00 |
| K nitrate | 5.00 | 5.00 | 5.00 |
| Na monofluorophosphate | 0.80 | 0.80 | 0.80 |
| PHB methyl ester | 0.15 | 0.15 | 0.15 |
| PHB propyl ester | 0.05 | 0.05 | 0.05 |
| Na saccharinate | 0.20 | 0.20 | 0.20 |
| Herbal flavour | 1.00 | 0.60 | 0.20 |
| Menthol particles from Example 1 | 0.125 | 0.56 | 1.00 |
| Ca carbonate | 35.00 | 35.00 | 35.00 |
| Silicon dioxide | 1.00 | 1.00 | 1.00 |
| Sodium dodecylsulfate (SDS) | 1.50 | 1.50 | 1.50 |
| Dist. water | ad 100.00 | ad 100.00 | ad 100.00 |

### F3. Ready-for-use mouthwash with fluoride

| | I (%) | II (%) | III (%) |
|---|---|---|---|
| Ethanol | 7.00 | 7.00 | 7.00 |
| Glycerol | 12.00 | 12.00 | 12.00 |
| Na fluoride | 0.05 | 0.05 | 0.05 |
| Pluronic F-127® (BASF, surface-active substance) | 1.40 | 1.40 | 1.40 |
| Na phosphate buffer, pH 7.0 | 1.10 | 1.10 | 1.10 |
| Sorbic acid | 0.20 | 0.20 | 0.20 |
| Na saccharinate | 0.10 | 0.10 | 0.10 |
| Thymol-wintergreen flavour | 1.00 | 0.60 | 0.20 |
| Menthol particles from Example 1 | 0.125 | 0.56 | 1.00 |
| Colouring | 0.01 | 0.01 | 0.01 |
| Dist. water | ad 100.00 | ad 100.00 | ad 100.00 |

### F4. Sugar-containing chewing gum

| | I (%) | II (%) | III (%) |
|---|---|---|---|
| Chewing gum base | 21.00 | 21.00 | 21.00 |
| Glucose syrup | 16.50 | 16.50 | 16.50 |
| Glycerol | 0.50 | 0.50 | 0.50 |
| Powdered sugar | 60.45 | 60.40 | 60.30 |
| Spearmint flavour | 1.40 | 1.00 | 0.50 |
| Menthol particles from Example 1 | 0.15 | 0.60 | 1.20 |

### F5. Sugar-free chewing gum

| | I (%) | II (%) | III (%) |
|---|---|---|---|
| Chewing gum base | 30.00 | 30.00 | 30.00 |
| Sorbitol, powder | 38.45 | 38.40 | 38.30 |
| Palatinite | 9.50 | 9.50 | 9.50 |
| Xylitol | 2.00 | 2.00 | 2.00 |
| Mannitol | 3.00 | 3.00 | 3.00 |
| Aspartame | 0.10 | 0.10 | 0.10 |
| Acesulfame K | 0.10 | 0.10 | 0.10 |
| Emulgum/Emulsifier | 0.30 | 0.30 | 0.30 |
| Sorbitol, 70 % in water | 14.00 | 14.00 | 14.00 |
| Glycerol | 1.00 | 1.00 | 1.00 |
| Cinnamon-menthol flavour | 1.40 | 1.00 | 0.50 |
| Menthol particles from Example 1 | 0.15 | 0.60 | 1.20 |

### F6. Chewing gum dragees, sugar-free

### Q1: Constituent chewing gum crude mass

| | I (%) | II (%) | III (%) |
|---|---|---|---|
| Chewing gum base | 37.00 | 37.00 | 37.00 |
| Sorbitol, powder | 50.50 | 50.50 | 50.50 |
| Aspartame | 0.20 | 0.20 | 0.20 |
| Plasticiser (Emulgum) | 0.50 | 0.50 | 0.50 |
| Acesulfame K | 0.20 | 0.20 | 0.20 |
| Sorbitol 70 % in water | 5.00 | 5.00 | 5.00 |
| Glycerol | 4.00 | 4.00 | 4.00 |
| Peppermint oil flavour (Optamint®, Symrise) | 1.60 | 1.60 | 1.60 |
| Menthol, spray-dried | 1.00 | 1.00 | 1.00 |

All the constituents of the chewing gum crude mass (Q1) were mixed, pressed into chewing gum strips and then fomed into individual cushion-shaped chewing gum tablets. The cushion-shaped chewing gum tablets were then wetted (gummed) with a 40 wt.% gum arabic solution in a rotating sugar-coating drum. The gummed cushion-shaped chewing gum tablets were subsequently coated with powder mixture A in a rotating sugar-coating drum, mixture A consisting of the menthol particles obtained according to the invention and a sugar substitute (selected from isomalt, sorbitol and mannitol; xylitol, maltitol and/or mannitol can alternatively be used; powdered gum arabic can optionally be used in addition). After sufficient drying with cold air, the cushion-shaped chewing gum tablets so coated were dried overnight. For the further application of the coating to the dried, coated cushion-shaped chewing gum tablets using coating solution B, 15 layers were first applied by means of sugar-coating; in the 16th layer, a mixture of constituent C and mixture B is applied. Further layers were then applied using mixture B until the total weight of the coating (Q2) was about 35 wt.% of the weight of the original cushion-shaped chewing gum tablets (Q1). In order to impart gloss to the chewing gum dragees, they were subsequently treated with a glossing agent which consisted of a mixture of equal parts by weight of carnauba wax and beeswax. When chewed, the ready-for-use chewing gum dragees produce in the mouth a new type of menthol taste which is very clear, intense and fresh.

### Q2: Constituent coating

### (the indicated parts by weight are based on the total weight of the coating (Q2) applied to the cushion-shaped chewing gum tablets (Q1); the total weight of Q2 was about 35 %, based on the mass Q1)

| | I (%) | II (%) | III (%) |
|---|---|---|---|
| **Mixture A** | | | |
| Isomalt | 0.20 | 0 | 0 |
| Sorbitol | 0 | 0.40 | 0 |
| Mannitol | 0 | 0 | 0.80 |
| Menthol particles from Example 1 | 0.20 | 0.60 | 1.00 |

| **Mixture B** | | | |
|---|---|---|---|
| Isomalt | 68.00 | 67.70 | 67.40 |
| Water | 26.7 | 26.6 | 26.5 |
| Gum arabic, 40% in water (this amount contains the quantity used for gumming) | 2.50 | 2.50 | 2.50 |
| Acesulfame K | 0.05 | 0.05 | 0.05 |
| Aspartame | 0.05 | 0.05 | 0.05 |
| Titanium dioxide | 1.50 | 1.50 | 1.50 |

| **Constituent C** | | | |
|---|---|---|---|
| Peppermint oil flavour (Optamint®, Symrise) | 0.80 | 0.60 | 0.20 |

### F7. Gelatine capsules for direct consumption

| | I (%) | II (%) | III (%) |
|---|---|---|---|
| Gelatine coating: | | | |
| Glycerol | 2.014 | 2.014 | 2.014 |
| Gelatine 240 Bloom | 7.91 | 7.91 | 7.91 |
| Sucralose | 0.065 | 0.065 | 0.065 |
| Allura red | 0.006 | 0.006 | 0.006 |
| Brilliant blue | 0.005 | 0.005 | 0.005 |
| | | | |

| Core composition: | | | |
|---|---|---|---|
| Vegetable oil triglyceride | 85.0 | 80.0 | 73.0 |
| Flavouring B | 4.0 | 6.0 | 10.0 |
| Menthol particles from Example 1 | 1.0 | 4.0 | 7.0 |

The present gelatine capsules were prepared according to WO 2004/050069.

### Flavouring B had the following composition (amounts are each in wt.%):

0.1 % neotame powder, 0.05 % aspartame, 29.3 % lemon oil, 29.3 % orange oil, 2.97 % sucralose, 2.28 % triacetin, 5.4 % diethyl tartrate, 12.1 % peppermint oil Yakima, 0.7 % ethanol, 3.36 % 2-hydroxyethylmenthyl carbonate, 3.0 % 2-hydroxypropylmenthyl carbonate, 0.27 % vanillin, 5.5 % D-limonene, 5.67 % L-menthyl acetate.

The gelatine capsule suitable for direct consumption had a diameter of 5 mm, and the weight ratio of core material to coating material was 90 : 10. The capsule opened in the mouth within a period of less than 10 seconds and dissolved completely within a period of less than 50 seconds.

### F8. Chewy sweet

| | | |
|---|---|---|
| Water | | 7.5 % |
| Sugar | refined sugar C4 | 41.2 % |
| Glucose syrup | dextrose 40 | 36.2 % |
| Hardened vegetable fat | melting point 32-36°C | 6.5 % |
| Lecithin | emulsifier (soya lecithin) | 0.3 % |
| Gelatine | porcine gelatine | 0.8 % |
| Fondant | type - S30 | 4.8 % |
| Lemon flavouring | | 0.6 % |
| Menthol particles from Example 1 | | 2.1 % |

### Notes on preparation:

a) allow the gelatine to swell in water (1.8 times the amount of gelatine) at 70°C for 2 hours;
b) boil the sugar, syrup, water, fat and lecithin at 123°C;
c) slowly mix the gelatine solution with the boiled mixture;
d) stir in the menthol particles and optional colouring;
e) adjust the resulting mass to a temperature of about 70°C on a cooling table, then add the fondant and expose to air for about 3 minutes on a drawing machine;
f) then cut and pack the chewy sweet mass.

When the chewy sweet is consumed, a strong menthol taste is perceived during chewing, the texture of the chewy sweet is pleasant.

### F9. Compressed product, with sugar or sugar-free

| | | |
|---|---|---|
| Dextrose (with sugar) or sorbitol (sugar-free) | | 98.5 - 98.8 % |
| Magnesium stearate | glidant | 1.0 % |
| Menthol particles from Example 1 | | 0.2 - 0.5 % |

All the constituents were mixed and the mixture was compressed in a suitable machine to form compressed products.

## Claims

1. Process for the preparation of spherical menthol particles, comprising the following steps:
- preparation of a menthol melt,
- metering of the menthol melt having a temperature in the range of from 42 to 60 °C into water having a temperature in the range of from 0 to 12 °C, the metering conditions being so chosen that menthol drops are temporarily present in the water after the metering, which menthol drops then solidify in the water to form the spherical menthol particles.

2. Process according to claim 1, wherein the menthol melt has a temperature in the range of from 45 to 55 °C during the metering, and/or
wherein the water has a temperature in the range of from 2 to 10 °C, preferably from 4 to 8 °C.

3. Process according to anyone of the preceding claims, wherein the menthol melt
(a) is metered into the water in the form of drops, or
(b) is metered into the water in the form of a stream, in such a manner that the stream breaks up in the water to form the drops.

4. Process according to any one of the preceding claims, wherein
(a) the menthol melt is metered into the water in such a manner that the menthol drops ascend upwards in the water, and/or
(b) the water is made to flow and the menthol melt is metered into the flowing water in such a manner that the menthol drops are transported by the flow of the water, preferably upwards, preferably upwards in vortex or spiral form.

5. Process according to any one of the preceding claims, wherein the solidified spherical menthol particles are left in the water at least until their surface temperature is not more than 20 °C.

6. Process according to any one of the preceding claims, wherein the water is placed in a vessel having a base and the menthol melt is metered into the water from the base.

7. Process according to any one of the preceding claims, wherein the solidified spherical menthol particles have a diameter in the range of from 0.3 to 10 mm, preferably from 0.5 to 8 mm, particularly preferably from 1 to 5 mm.

8. Process according to any one of the preceding claims, wherein the spherical menthol particles are separated from the water and dried to a moisture content of less than 1 wt.%, preferably less than 0.5 wt.% based on the total weight of the menthol particles.

9. Process according to any one of the preceding claims, wherein the water is placed in a vessel having an overflow, and the spherical menthol particles are discharged from the vessel through the overflow.

## Patentansprüche

1. Verfahren zur Herstellung von kugelförmigen Mentholpartikeln, umfassend die folgenden Schritte:
- Herstellung einer Mentholschmelze,
- Dosieren der Mentholschmelze mit einer Temperatur im Bereich von 42 bis 60 °C in Wasser mit einer Temperatur im Bereich von 0 bis 12 °C, wobei die Dosierbedingungen so gewählt werden, dass nach der Dosierung vorübergehend Mentholtropfen im Wasser vorhanden sind, die dann im Wasser zu den kugelförmigen Mentholpartikeln erstarren.

2. Verfahren nach Anspruch 1, wobei die Mentholschmelze während der Dosierung eine Temperatur im Bereich von 45 bis 55 °C und/oder wobei das Wasser eine Temperatur im Bereich von 2 bis 10 °C, vorzugsweise von 4 bis 8 °C, hat.

3. Verfahren nach einem der vorstehenden Ansprüche, bei dem die Mentholschmelze mit einer Temperatur im Bereich von 2 bis 10 °C, vorzugsweise 4 bis 8 °C, geschmolzen wird.
(a) in Form von Tropfen in das Wasser dosiert wird, oder
(b) wird in Form eines Stroms in das Wasser dosiert, und zwar so, dass der Strom im Wasser aufbricht und die Tropfen bildet.

4. Verfahren nach einem der vorstehenden Ansprüche, wobei
(a) die Mentholschmelze in das Wasser dosiert wird, so dass die Mentholtropfen nach oben aufsteigen, und/oder
(b) das Wasser zum Fließen gebracht und die Mentholschmelze so in das fließende Wasser dosiert wird, dass die Mentholtropfen durch den Wasserstrom vorzugsweise nach oben, vorzugsweise in Wirbel- oder Spiralform nach oben transportiert werden.

5. Verfahren nach einem der vorstehenden Ansprüche, bei dem die erstarrten kugelförmigen Mentholpartikel mindestens so lange im Wasser belassen werden, bis ihre Oberflächentemperatur nicht mehr als 20 °C beträgt.

6. Verfahren nach einem der vorstehenden Ansprüche, bei dem das Wasser in ein Gefäß mit einem Boden eingebracht und die Mentholschmelze vom Boden aus in das Wasser dosiert wird.

7. Verfahren nach einem der vorstehenden Ansprüche, bei dem die erstarrten kugelförmigen Mentholpartikel einen Durchmesser im Bereich von 0,3 bis 10 mm, vorzugsweise 0,5 bis 8 mm, besonders bevorzugt 1 bis 5 mm, aufweisen.

8. Verfahren nach einem der vorstehenden Ansprüche, wobei die kugelförmigen Mentholpartikel vom Wasser getrennt und auf einen Feuchtigkeitsgehalt von weniger als 1 Gew.-%, vorzugsweise weniger als 0,5 Gew.-%, bezogen auf das Gesamtgewicht der Mentholpartikel, getrocknet werden.

9. Verfahren nach einem der vorstehenden Ansprüche, wobei das Wasser in ein Gefäß mit einem Überlauf eingebracht wird und die kugelförmigen Mentholpartikel durch den Überlauf aus dem Gefäß ausgetragen werden.

## Revendications

1. Procédé de préparation de particules sphériques de menthol, comprenant les étapes suivantes :
- préparation d'un fondant au menthol,
- dosage de la masse fondue de menthol ayant une température dans la plage de 42 à 60 °C dans de l'eau dont la température est comprise entre 0 et 12 °C, les conditions de dosage étant choisies de telle sorte que des gouttes de menthol soient temporairement présentes dans l'eau après le dosage, lesquelles gouttes de menthol se solidifient ensuite dans l'eau pour former les particules sphériques de menthol.

2. Procédé selon la revendication 1, dans lequel la masse fondue de menthol a une température comprise entre 45 et 55 °C pendant le dosage, et/ou dans laquelle l'eau a une température dans la plage de 2 à 10 °C, de préférence de 4 à 8 °C.

3. Procédé selon l'une quelconque des revendications précédentes, dans lequel le menthol fond en fusion
a) est dosée dans l'eau sous forme de gouttes, ou
b) est mesurée dans l'eau sous la forme d'un cours d'eau, de telle sorte que le cours d'eau se fragmente dans l'eau pour former les gouttes.

4. Procédé selon l'une quelconque des revendications précédentes, dans lequel
a) la masse fondue de menthol est dosée dans l'eau, et/ou
b) l'eau est amenée à s'écouler et la masse fondue de menthol est dosée dans l'eau de telle sorte que les gouttes de menthol soient transportées par l'écoulement de l'eau, de préférence vers le haut, de préférence vers le haut sous forme de vortex ou de spirale.

5. Procédé selon l'une quelconque des revendications précédentes, dans lequel les particules de menthol sphériques solidifiées sont laissées dans l'eau au moins jusqu'à ce que leur température de surface ne dépasse pas 20 °C.

6. Procédé selon l'une quelconque des revendications précédentes, dans lequel l'eau est placée dans un récipient ayant une base et la masse fondue de menthol est dosée dans l'eau depuis la base.

7. Procédé selon l'une quelconque des revendications précédentes, dans lequel les particules de menthol sphériques solidifiées ont un diamètre compris entre 0,3 et 10 mm, de préférence entre 0,5 et 8 mm, en particulier de 1 à 5 mm.

8. Procédé selon l'une quelconque des revendications précédentes, dans lequel les particules sphériques de menthol sont séparées de l'eau et séchées jusqu'à une teneur en humidité inférieure à 1% en poids, de préférence inférieure à 0,5% en poids par rapport au poids total des particules de menthol.

9. Procédé selon l'une quelconque des revendications précédentes, dans lequel l'eau est placée dans un récipient ayant un trop-plein, et les particules sphériques de menthol sont évacuées du récipient par le trop-plein.
